# EUROPÄISCHE PATENTANMELDUNG

(11) **EP 2 025 657 A2**
(43) Veröffentlichungstag der Anmeldung: **18.02.2009**
(21) Anmeldenummer: 08169278.2
(22) Anmeldetag: 08.01.2004
(51) Int. Cl.: C04B 35/524, C04B 35/63, C04B 35/632, C04B 35/634, C12N 5/00, C07K 16/00, A61L 27/34, D06C 7/04, B01D 71/02, B01J 37/08, B01J 27/20, C04B 41/00, C04B 41/45, C04B 41/50, C04B 41/51, C04B 41/88, C04B 38/00, C04B 38/06, C04B 38/04, B01J 21/18, B01J 37/00, B01J 37/06

(54) **Verfahren zur Herstellung von kohlenstoffbasierten Formkörpern und deren Verwendung als Zellkulturträger- und Aufzuchtsysteme**

(30) Priorität: 31.07.2003 DE 10335131
(62) Teilanmeldung aus: 04700670.5
(71) Anmelder: CINVENTION AG, 65203 Wiesbaden (DE)
(72) Erfinder: Rathenow, Jörg, 65207, Wiesbaden (DE); Asgari, Sohéil, 65193, Wiesbaden (DE); Kunstmann, Jürgen, 65812, Bad Soden (DE)
(74) Vertreter: Maiwald Patentanwalts GmbH

(57) **Zusammenfassung**

Die vorliegende Erfindung betrifft Verfahren zur Herstellung poröser kohlenstoffbasierter Formkörper durch Karbonisierung organischer Polymermaterialien, wobei der kohlenstoffbasierte Formkörper nach der Karbonisierung zur Erzeugung von Poren teiloxidiert wird. Darüber hinaus betrifft die vorliegende Erfindung nach einem der genannten Verfahren hergestellte poröse Formkörper sowie deren Verwendung, insbesondere als Zellkulturträger- und/oder Aufzuchtsysteme.

## Beschreibung

Die vorliegende Erfindung betrifft Verfahren zur Herstellung von kohlenstoffbasierten Formkörpern. Insbesondere betrifft die vorliegende Erfindung Verfahren zur Herstellung poröser kohlenstoffbasierter Formkörper durch Karbonisieren von organischen Polymermaterialien, die mit nichtpolymeren Füllstoffen vermischt sind, und anschließendes Herauslösen der Füllstoffe aus dem karbonisierten Formkörper. In einer weiteren Ausführungsform betrifft die vorliegende Erfindung ein Verfahren zur Herstellung poröser kohlenstoffbasierter Formkörper, durch Karbonisieren von organischen Polymermaterialien mit polymeren Füllstoffen, die bei der Karbonisierung im Wesentlichen vollständig abgebaut werden. Ferner betrifft die vorliegende Erfindung ein Verfahren zur Herstellung poröser kohlenstoffbasierter Formkörper durch Karbonisierung organischer Polymermaterialien, wobei der kohlenstoffbasierte Formkörper nach der Karbonisierung zur Erzeugung von Poren teiloxidiert wird. Darüber hinaus betrifft die vorliegende Erfindung nach einem der genannten Verfahren hergestellte poröse Formkörper sowie deren Verwendung, insbesondere als Zellkulturträger- und/oder Aufzuchtsysteme.

Kohlenstoff ist aufgrund der Variabilität seiner Eigenschaften in allen Bereichen der Werkstofftechnik ein vielseitig einsetzbares Material. Kohlenstoffbasierte Werkstoffe finden Anwendungen im Maschinenbau, Fahrzeugbau, wie auch in Medizintechnik und Verfahrenstechnik. Aus der DE 35 28 185 ist ein Verfahren zur Herstellung von hochfestem, hochdichten Kohlenstoffmaterialien aus speziellen gepulverten kohlenstoffhaltigen Rohmaterialien ohne Verwendung eines Bindemittels beschrieben.

Die DE 198 23 507 beschreibt Verfahren zur Herstellung von kohlenstoffbasierten Formkörpern durch Karbonisierung biogener Vorprodukte aus natürlichen Pflanzenfasern oder Holzprodukt. Die DE 100 11 013 und die EP 0 543 752 beschreiben Verfahren zur Herstellung kohlenstoffhaltiger Materialien durch Karbonisierung bzw. Pyrolyse von geschäumten Ausgangspolymeren wie Polyacrylnitril oder Polyurethan. Die so erhaltenen Kohlenstoffschäume werden als Hochtemperaturisolatoren im Ofenanlagen oder Reaktorbau oder zur Schalldämmung im Hochtemperaturbereich verwendet. Auch die US 3,342,555 beschreibt ein Verfahren zur Herstellung leichten porösen Kohlenstoffs durch Karbonisierung von geschäumten Polymeren auf Basis von Phenolaldehydharzen des Resol- oder Novolactyps.

Die genannten Verfahren des Standes der Technik zur Erzeugung poröser Kohlenstoffformkörper weisen den Nachteil auf, dass die durch Karbonisierung geschäumter Polymere erhaltenen Formkörper oft eine sehr geringe mechanische Stabilität aufweisen, was deren Verwendung unter mechanischen Belastungsbedingungen nahezu unmöglich macht. Ferner lassen sich Porengröße und Porenvolumen in diesen Formkörpern nicht hinreichend genau einstellen um diese beispielsweise für biotechnologische Anwendungen, wie etwa als orthopädische Implantate, verwendbar zu machen.

Es besteht daher ein fortwährender Bedarf nach neuen und verbesserten Verfahren zur Herstellung von porösen kohlenstoffhaltigen Formkörpern.

Es ist eine Aufgabe der vorliegenden Erfindung ein einfaches unter wirtschaftlichen Bedingungen durchführbares Verfahren zur Herstellung poröser kohlenstoffbasierter Formkörper anzugeben.

Eine weitere Aufgabe der vorliegenden Erfindung ist es, Verfahren zur Herstellung poröser kohlenstoffbasierter Formkörper anzugeben, die durch Variation einfacher Prozessparameter eine gezielte Einstellung der Porosität, insbesondere des Porenvolumens und der Porendurchmesser in reproduzierbarer Weise erlauben.

Eine weitere Aufgabe der vorliegenden Erfindung ist es Herstellungsverfahren für poröse kohlenstoffbasierte Formkörper zur Verfügung zu stellen, welche die maßgeschneiderte Herstellung entsprechender Formkörper in einer Vielzahl von Formen und Abmessungen ermöglichen.

Ferner ist es eine Aufgabe der vorliegenden Erfindung Einsatzgebiete und Anwendungen der erfindungsgemäßen kohlenstoffbasierten Formkörper anzugeben.

Die Lösung der erfindungsgemäßen Aufgaben ergibt sich durch die Verfahren und danach herstellbaren Formkörper sowie die Verwendungen gemäß den unabhängigen Ansprüchen. Bevorzugte Ausführungsformen sind in den jeweiligen abhängigen Ansprüchen aufgeführt.

Im Allgemeinen stellt die vorliegende Erfindung Verfahren zur Verfügung, bei welchen poröse kohlenstoffbasierte Formkörper durch Karbonisieren von Halbzeugformteilen aus organischen Polymermaterialien hergestellt werden, wobei die Porosität des Formkörpers während oder im Anschluss an die Pyrolyse erzeugt wird.

In einer ersten Ausführungsform der vorliegenden Erfindung wird ein Verfahren zur Herstellung von porösen kohlenstoffbasierten Formkörpern zur Verfügung gestellt, welches die folgenden Schritte umfasst:
- Mischen von zu Kohlenstoff karbonisierbaren, organischen Polymermaterialien mit nichtpolymeren Füllstoffen;
- Herstellen eines Halbzeugformteils aus der Mischung;
- Karbonisieren des Halbzeugformteils in nichtoxidierender Atmosphäre bei erhöhter Temperatur, wobei ein kohlenstoffbasierter Formkörper erhalten wird;
- Herauslösen der Füllstoffe aus dem karbonisierten Formkörper mit geeigneten Lösemitteln.

Gemäß dieser Ausführungsformen des erfindungsgemäßen Verfahrens werden in einem ersten Schritt die zu Kohlenstoff karbonisierbaren organischen Polymermaterialien mit nichtpolymeren Füllstoffen vermischt oder vermengt. Dies kann prinzipiell durch den Fachmann bekannte geeignete Mischverfahren erfolgen, wie beispielsweise Trockenvermischung von Polymerpellets mit Füllstoffpulvern oder Granulaten, Einmischen von Füllstoffen in die Polymerschmelze oder Vermischung der Füllstoffe mit Polymerlösungen oder -Suspensionen.

Als nichtpolymere Füllstoffe sind alle Substanzen geeignet, die unter Karbonisierungsbedingungen im Wesentlichen stabil sind und sich nach der Karbonisierung aus dem kohlenstoffbasierten Formkörper mit geeigneten Lösemitteln entfernen lassen. Weiterhin sind nichtpolymere Füllstoffe geeignet, die unter Karbonisierungsbedingungen zu Lösemittel-löslichen Substanzen umgewandelt werden.

Bevorzugte Füllstoffe sind ausgewählt aus anorganischen Metallsalzen, insbesondere der Salze von Alkali- und/oder Erdalkalimetallen, vorzugsweise Alkali- oder Erdalkalimetallcarbonate, -Sulfate, -Sulfite, - Nitrate, -Nitrite, -Phosphate, - Phosphite, -Halogenide, -Sulfide, -Oxide, sowie Mischungen dieser. Weitere geeignete Füllstoffe werden ausgewählt aus organischen Metallsalzen, vorzugsweise solche der Alkali-, Erdalkali- und/oder Übergangsmetalle, insbesondere deren Formiate, Azetate, Propionate, Aleate, Malate, Oxalate, Tartrate, Zitrate, Benzoate, Salicylate, Phtalate, Stearate, Phenolate, Sulfonate, Aminsalze sowie deren Mischungen.

Geeignete Lösemittel zum Herauslösen der Füllstoffe aus dem karbonisierten Formkörper sind beispielsweise Wasser, insbesondere heißes Wasser, verdünnte oder konzentrierte anorganische oder organische Säuren, Laugen und dergleichen. Geeignete anorganische Säuren sind in verdünnter oder konzentrierter Form Salzsäure, Schwefelsäure, Phosphorsäure, Salpetersäure sowie verdünnte Flusssäure.

Geeignete Laugen sind z.B. Natronlauge, Ammoniaklösung, Karbonatlösungen, aber auch organische Aminlösungen.

Geeignete organische Säuren sind Ameisensäure, Essigsäure, Trichlormethansäure, Trifluormethansäure, Zitronensäure, Weinsäure, Oxalsäure und Mischungen davon.

Die Füllstoffe können im Wesentlichen vollständig oder teilweise aus dem karbonisierten Formkörper herausgelöst werden, je nach Art und Anwendungsdauer des Lösemittels. Bevorzugt ist das im Wesentlichen vollständige Lösen der Füllstoffe.

Je nach Anwendungszweck und gewünschter Porosität bzw. Porendimension können die Füllstoffe in geeigneten Korngrößen eingesetzt werden. Besonders bevorzugt sind Pulver oder granulatförmige Füllstoffe mit mittleren Partikelgrößen von 3 Angström bis 2 mm, besonders bevorzugt 1 nm bis 500 µm und insbesondere bevorzugt 10 nm bis 100 µm.

Geeignete Partikelgrößen der nichtpolymeren Füllstoffe wird der Fachmann in Abhängigkeit von der gewünschten Porosität und der gewünschten Porendimensionen des fertigen karbonisierten Formkörpers auswählen.

Weiterhin geeignete Lösemittel zum Herauslösen der Füllstoffe sind organische Lösungsmittel, wie Methanol, Ethanol, N-Propanol, Isopropanol, Butoxydiglycol, Butoxyethanol, Butoxyisopropanol, Butoxypropanol, n-Butyl-Alkohol, t-Butyl-Alkohol, Butyleneglycol, Butyloctanol, Diethylenglycol, Dimethoxydiglycol, Dimethylether, Dipropylenglycol, Ethoxydiglycol, Ethoxyethanol, Ethylhexandiol, Glycol, Hexanediol, 1,2,6-Hexanetriol, Hexylalkohol, Hexylenglycol, Isobutoxypropanol, Isopentyldiol, 3-Methoxybutanol, Methoxydiglycol, Methoxyethanol, Methoxyisopropanol, Methoxymethylbutanol, Polypropylenglycol, Methylal, Methyl-Hexylether, Methylpropanediol, Neopentylglycol, Polyethylenglycol, Pentylenglycol, Propanediol, Propylenglycol, Propylenglycol-Butylether, Propylenglycol-Propylether, Tetrahydrofuran, Trimethylhexanol, Phenol, Benzol, Toluol, Xylol; als auch Wasser, ggf. im Gemisch mit Dispersionshilfsmitteln, sowie Mischungen der obengenannten.

Auch Mischungen organischer Lösemittel mit Wasser und/oder anorganischen und/oder organischen Säuren können in bestimmten Ausführungsformen der vorliegenden Erfindung eingesetzt werden um die nichtpolymeren Füllstoffe aus dem karbonisierten Formkörper herauszulösen.

In einer zweiten Ausführungsform der Erfindung wird ein Verfahren zur Herstellung von porösen kohlenstoffbasierten Formkörpern zur Verfügung gestellt, umfassend die folgenden Schritte:
- Mischen von zu Kohlenstoff karbonisierbaren organischen Polymermaterialien mit polymeren Füllstoffen;
- Herstellen eines Halbzeugformteils aus der Mischung;
- Karbonisieren des Halbzeugformteils in nichtoxidierender Atmosphäre bei erhöhter Temperatur, wobei die polymeren Füllstoffe im Wesentlichen vollständig abgebaut werden.

Gemäß dieser Ausführungsform der Erfindung werden die Poren im kohlenstoffbasierten Formkörper während der Karbonisierung dadurch erzeugt, dass polymere Füllstoffe in die zu Karbonisierenden organischen Polymermaterialien eingebaut werden, die unter Karbonisierungsbedingungen im Wesentlichen vollständig abgebaut werden.

Ohne auf eine bestimmte Theorie festgelegt werden zu wollen hat sich gezeigt, dass bestimmte polymere Füllstoffe, insbesondere gesättigte aliphatische Kohlenwasserstoffe unter den Bedingungen der Karbonisierung, d. h. hohe Temperaturen und Sauerstoffausschluss, im Wege von crack-analogen Verfahren im Wesentlichen vollständig zu flüchtigen Kohlenwasserstoffen wie Methan, Ethan und dergleichen abgebaut werden können, die dann aus dem porösen Kohlenstoffgerüst des karbonisierten Formkörpers während der Pyrolyse bzw. Karbonisierung entweichen.

Geeignete polymere Füllstoffe können ausgewählt werden aus gesättigten, verzweigten oder unverzweigten aliphatischen Kohlenwasserstoffen, welche Homo- oder Copolymere sein können. Bevorzugt sind hierbei Polyolefine wie Polyethylen, Polypropylen, Polybuten, Polyisobuten, Polypenten sowie deren Copolymere und Mischungen.

Die polymeren Füllstoffe werden in einem ersten Schritt mit den karbonisierbaren organischen Polymermaterialien vermischt. Dies kann nach dem Fachmann prinzipiell bekannten Verfahren des Standes der Technik erfolgen, beispielsweise Vermischung von Polymerpellets bzw. -granalien, Einmischen polymerer Füllstoffe in Schmelzen aus karbonisierbaren organischen Polymermaterialien oder Suspensionen oder Lösungen dieser Polymermaterialien, Koextrusion der polymeren Füllstoffe mit den karbonisierbaren organischen Polymermaterialien und dergleichen.

Durch geeignete Wahl des Molgewichts, der Kettenlänge und/oder des Verzweigungsgrades der polymeren Füllstoffe können die im karbonisierten Formkörper erzeugten Poren geeignet dimensioniert bzw. in weiten Grenzen variiert werden. Auch können die polymeren Füllstoffe in Form dünner Fasern verwendet werden, die bei der Karbonisierung geeignet dimensionierte Porengänge bilden. Die Porosität ist durch Wahl des Faserdurchmessers und der Faserlänge einstellbar, wobei größere Faserdurchmesser und -längen eine größere Porosität bedingen. Hierbei können auch durch geeignete Mischung der verwendeten Fasern gewünschte Zwischeneffekte realisiert werden, oder auch asymmetrische Porositätsverteilungen und Texturen der Formkörper.

Diese Ausführungsform des erfindungsgemäßen Verfahrens unter Verwendung polymerer Füllstoffe als Porenbildner ist insbesondere geeignet für poröse Formkörper mit kleinen Porengrößen im Nano- bis Mikrometerbereich, insbesondere mit Porengrößen von 3 Angström bis 2 mm, besonders bevorzugt 1 nm bis 500 µm und insbesondere bevorzugt 10 nm bis 100 µm.

In einer bevorzugten Ausführungsform dieses Verfahrens wird der karbonisierte Formkörper nach der Karbonisierung mit geeigneten Oxidations- und/oder Reduktionsmitteln nachbehandelt, um die Porengrößen weiter zu modifizieren. Auch ein nachträgliches Verdichten oder Verschliessen der Poren, beispielsweise durch CVD/CVI-Verfahren unter Abscheidung geeigneter organischer oder Anorganischer Precursor kann erfindungsgemäß verwendet werden, um Formkörper mit gewünschten Eigenschaften "maßzuschneidern".

Gemäß einer dritten Ausführungsform des erfindungsgemäßen Verfahrens wird ein Verfahren zur Herstellung von porösen kohlenstoffbasierten Formkörpern angegeben, welches die folgenden Schritte umfasst:
- Herstellen eines Halbzeugformteils aus karbonisierbaren organischen Polymermaterialien;
- Karbonisieren des Halbzeugformteils in nichtoxidierender Atmosphäre bei erhöhter Temperatur, wobei ein kohlenstoffbasierter Formkörper erhalten wird; und
- Teiloxidation des karbonisierten Formkörpers zur Erzeugung von Poren.

Gemäß dieser Ausführungsform des erfindungsgemäßen Verfahrens wird durch Karbonisierung geeigneter Polymermaterialien ein Formkörper gebildet, und nach der Karbonisierung mittels geeigneter Oxidationsmittel im karbonisierten Formkörper Porosität erzeugt und/oder vergrößert, indem durch partielle Oxidation des Kohlenstoffs Poren in den kohlenstoffbasierten Formkörper "gebrannt" werden.

Zur Erzeugung von Poren in dem kohlenstoffbasierten Formkörper sind im Wesentlichen alle zur Oxidation von Kohlenstoffmaterialien geeigneten Oxidationsverfahren und Oxidationsmittel geeignet.

Bevorzugt ist die Behandlung des karbonisierten Formkörpers bei erhöhter Temperatur in oxidierenden Gasatmosphären. Geeignete Oxidationsmittel für die Teiloxidation in oxidierender Gasphase sind Luft, Sauerstoff, Kohlenmonoxid, Kohlendioxid, Stickstoffoxide und ähnliche Oxidationsmittel. Diese gasförmigen Oxidationsmittel können mit inerten Gasen wie Edelgasen, insbesondere Argon, oder auch Stickstoff gemischt werden und geeignete Volumenkonzentrationen des Oxidationsmittels exakt eingestellt werden. Durch Reaktion mit diesen Oxidationsmitteln werden in den porösen Formkörper im Wege einer partiellen Oxidation Löcher bzw. Poren eingebrannt.

Bevorzugt wird die Teiloxidation bei erhöhten Temperaturen durchgeführt, insbesondere im Bereich von 50°C bis 800°C.

In einem besonders bevorzugten Verfahren dieser Ausführungsform erfolgt die Teiloxidation durch Behandeln des Formkörpers mit, ggf. strömender, Luft bei Raumtemperatur oder darüber.

Neben der partiellen Oxidation des Formkörpers mit gasförmigen Oxidationsmitteln können auch flüssige Oxidationsmittel verwendet werden, wie beispielsweise konzentrierte Salpetersäure, die in geeigneter Weise auf den Formkörper aufgebracht wird. Auch hierbei kann es bevorzugt sein, die konzentrierte Salpetersäure bei Temperaturen oberhalb von Raumtemperatur mit dem karbonisierten Formkörper in Kontakt zu bringen um eine oberflächliche oder tiefergehende Porenerzeugung zu gewährleisten.

Die obengenannten Verfahren der Erzeugung von Poren können erfindungsgemäß auch miteinander kombiniert werden. So ist es erfindungsgemäß möglich, neben löslichen Füllstoffen zusätzlich polymere Füllstoffe zu verwenden, die unter Karbonisierungsbedingungen flüchtig sind oder zu flüchtigen Stoffen abgebaut werden. Auf diese Weise lassen sich die aus den Füllstoffen erzeugten gröberen Poren mit den Mikro- oder Nanoporen der polymeren Füllstoffe zu ansiotropen Porenverteilungen verknüpfen. Ferner können zusätzlich zur Porenerzeugung mit Füllstoffen und/oder polymeren Feststoffen die vorhandenen Poren auch durch partielle Oxidation erweitern, miteinander verknüpfen oder modifizieren.

Weiterhin besteht die Möglichkeit die Poren z.B. durch Behandlung mit flüssigkristallinen Teerpechen, zu verschließen und ggf. einer erneuten Temperaturbehandlung zu unterziehen. Durch die Karbonisierung können so hochgeordnete, kristalline Bereiche erzielt werden. Durch Kombination der erfindungsgemäßen Verfahren lassen sich z.B. asymmetrische und symmetrische Gradientenwerkstoffe erhalten.

### ORGANISCHES POLYMERMATERIAL

In allen drei genannten Ausführungsformen des erfindungsgemäßen Verfahrens werden als zu Kohlenstoff karbonisierbares organisches Polymermaterial solche Materialien eingesetzt, die unter Karbonisierungsbedingungen, d. h. bei erhöhter Temperatur und in einer im Wesentlichen sauerstofffreien Atmosphäre, Kohlenstoffmaterialien aus amorphem, teilkristallinem und/oder kristallinem symmetrischem oder asymmetrischen Material zurückbleiben.

Ohne auf eine bestimmte Theorie festgelegt werden zu wollen hat sich gezeigt, dass hierzu insbesondere ungesättigte, verzweigte aliphatische Kohlenwasserstoffe, verzweigte oder unverzweigte, vernetzte oder unvernetzte aromatische oder teilaromatische Kohlenwasserstoffe, sowie substituierte Derivate davon geeignet sind. Ungesättigte Kohlenwasserstoffe, insbesondere aromatische Kohlenwasserstoffe werden unter Karbonisierungsbedingungen in der Regel zu Graphit-ähnlichen vernetzten Sechsringstrukturen gebaut, welche das Grundgerüst des carbonisierten Formkörpers bilden.

Auch gesättigte aliphatische und/oder aromatische Kohlenwasserstoffe mit Heteroatomanteilen, wie Ether, Urethane, Amide und Amine und dergleichen sind als karbonisierbare organische Polymermaterialien oder in Mischungen mit anderen aliphatischen oder aromatischen ungesättigten Kohlenwasserstoffen geeignet in den erfindungsgemäßen Verfahren.

Bevorzugt werden in den erfindungsgemäßen Verfahren carbonisierbare organische Polymermaterialien ausgewählt aus: Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phtalat), Alkydharz, Chlorkautschuk, Epoxidharz, Acrylatharz, Phenolharz, Aminharz, Melaminharz, Alkylphenolharze, epoxidierte aromatische Harze, Teer, teerartige Materialien, Teerpech, flüssigkristalines Teerpech, Bitumen, Stärke, Zellulose, Schellack, organische Materialien aus nachwachsenden Rohstoffen sowie deren Copolymere, Mischungen und Kombinationen dieser Homo- oder Copolymere.

Die karbonisierbaren organischen Polymermaterialien können ferner übliche Additive wie weitere insbesondere unlösliche Füllstoffe, Weichmacher, Gleitmittel, Flammschutzmittel, Glas, Glasfasern, Kohlefasern, Baumwolle, Gewebe, Metallpulver, Metallverbindungen, Silizium, Siliziumoxide, Zeolithe, Titanoxide, Zirkonoxide, Aluminiumoxide, Aluminosilikate, Talkum, Graphit, Russ, Tonmaterialien, Phyllosilikate und dergleichen enthalten. Insbesondere faserartige Materialien aus Cellulose, Baumwolle, Textilgeweben, Glasfasern, Kohlefasern und dergleichen als Polymeradditive sind in bevorzugten Ausführungsformen der vorliegenden Erfindung dazu geeignet die mechanischen Eigenschaften der erzeugten porösen Formkörper zu verbessern.

Die Herstellung der Halbzeugformteile gemäß den Verfahren der vorliegenden Erfindung kann mittels üblichen und dem Fachmann bekannten Formgebungsverfahren für Polymermaterialien erfolgen. Geeignete Formgebungsverfahren sind Gussverfahren, Extrusionsverfahren, Pressverfahren, Spritzgussverfahren, Koextrusionsblasformen oder andere übliche Formgebungsverfahren, beispielsweise auch Wickelverfahren oder Strangwickelverfahren unter Verwendung flächiger Ausgangsmaterialien.

### KARBONISIERUNG

Die Karbonisierung erfolgt bei den erfindungsgemäßen Verfahren in einer im Wesentlichen sauerstofffreien bzw. oxidationsmittelfreien Atmosphäre. Geeignete Karbonisierungsatmosphären sind beispielsweise Schutzgas, vorzugsweise Stickstoff und/oder Argon, Edelgase, SiF₆ und Mischungen dieser Schutzgase. Gegebenenfalls können diese Schutzgasatmosphären bei Unterdruck oder Überdruck angewendet werden. Auch die Karbonisierung im Vakuum kann bei den erfindungsgemäßen Verfahren vorteilhaft eingesetzt werden.

Ferner kann es bevorzugt sein, des Inertgasatmosphäre reaktive Gase beizumischen. Bevorzugte reaktive Gase hierfür sind nichtoxidierende Gase wie Wasserstoff, Ammoniak, C₁-C₆ gesättigte aliphatische Kohlenwasserstoffe wie Methan, Ethan, Propan und Butan, Mischungen dieser, und dergleichen.

Geeignete Temperaturen für den Karbonisierungsschritt liegen im Bereich von 200°C bis zu 4000°C oder mehr. Je nach gewählter Temperatur im Karbonisierungsschritt und der Art des verwendeten Polymermaterials lassen sich kohlenstoffhaltige Formkörper erzeugen, deren Basismaterial in seiner Struktur von amorph bis zu geordnet kristallinen graphitartigen Strukturen oder Mischungen beider Werkstoffe reicht.

Der Fachmann wird in Abhängigkeit von den spezifischen temperaturabhängigen Eigenschaften der verwendeten Polymermaterialien bzw. Ausgangsmaterialmischungen eine geeignete Temperatur, geeignete Atmosphäre und geeignete Druckbedingungen auswählen.

Die Atmosphäre beim Karbonisierungsschritt der erfindungsgemäßen Verfahren ist im Wesentlichen frei von Sauerstoff, vorzugsweise mit O₂ gehalten unter 10 ppm, besonders bevorzugt unter 1 ppm. Bevorzugt ist die Verwendung von Wasserstoff- oder Inertgasatmosphären, beispielsweise aus Stickstoff, Edelgasen wie Argon, Neon, sowie beliebige andere inerte, nicht mit Kohlenstoff reagierende Gase oder Gasverbindungen sowie deren Mischungen. Besonders bevorzugt ist Stickstoff.

Der Karbonisierungsschritt wird vorzugsweise in einem diskontinuierlichen Verfahren in geeigneten Öfen stattfinden, kann aber auch in kontinuierlichen Ofenprozessen durchgeführt werden, was gegebenenfalls auch bevorzugt sein kann.

Die Halbzeugformteile werden dabei auf einer Seite dem Ofen zugeführt und am anderen Ende des Ofens wieder austreten. In bevorzugten Ausführungsformen kann das Halbzeugformteil im Ofen auf einer Lochplatte, einem Sieb oder dergleichen aufliegen, so dass durch den Polymerfilm während der Pyrolyse bzw. Karbonisierung Unterdruck angelegt werden kann. Dies ermöglicht einerseits eine einfache Fixierung der Implantate im Ofen, andererseits auch eine Absaugung und optimale Durchströmung der Halbzeugformteile mit Inertgas während der Karbonisierung.

Der Ofen kann durch entsprechende Inertgasschleusen in einzelne Segmente unterteilt werden, in welchen nacheinander ein oder mehrere Karbonisierungsschritte, gegebenenfalls bei unterschiedlichen Karbonisierungsbedingungen wie zum Beispiel unterschiedlichen Temperaturstufen, verschiedenen Inertgasen bzw. Vakuum durchgeführt werden können. Ferner können in entsprechenden Segmenten des Ofens gegebenenfalls auch Nachbehandlungs-, Aktivierungs- oder Zwischenbehandlungsschritte erfolgen, wie beispielsweise partielle Oxidation, Reduktion oder auch Imprägnierung mit Metallsalzlösungen und dergleichen.

Alternativ hierzu kann die Karbonisierung in einem geschlossenen Ofen durchgeführt werden, was insbesondere dann bevorzugt ist, wenn die Karbonisierung im Vakuum durchgeführt werden soll. Je nach verwendetem karbonisierbarem organischem Polymermaterial bzw. eingesetzten Füllstoffen tritt während des Karbonisierungsschritts in den erfindungsgemäßen Verfahren eine Gewichtsabnahme des Materials von ca. 5 % bis 95 %, vorzugsweise von ca. 40 % bis 90 %, insbesondere 50 % bis 70 % auf.

### NACHBEHANDLUNG

In bevorzugten Ausführungsformen der Erfindung werden die physikalischen und chemischen Eigenschaften der kohlenstoffbasierten Formkörper bzw. der erzeugten Poren nach der Karbonisierung durch geeignete Nachbehandlungsschritte weiter modifiziert und dem jeweils gewünschten Verwendungszweck angepasst.

Geeignete Nachbehandlungen sind beispielsweise reduzierende oder oxidative Nachbehandlungsschritte, bei welchem die porösen Formkörper mit geeigneten Reduktionsmitteln und/oder Oxidationsmitteln wie Wasserstoff, Kohlendioxid, Stickstoffoxide wie N₂O, Wasserdampf, Sauerstoff, Luft, Salpetersäure und dergleichen sowie ggf. Mischungen dieser behandelt werden.

Weiterhin können Beschichtungen der Oberflächen erfolgen, die einseitig oder beidseitg erfolgen können. Geeignete Beschichtungsmaterialien können z.B. die oben genannten organischen Polymermaterialien sein, die gegebenenfalls nach Auftragung einem weiteren Karbonisierungs- bzw. Pyrolyseschritt unterzogen werden, um asymmetrische Texturen im Formkörper zu erzielen. Auch die Beschichtung mit anorganischen Stoffen, bioverträglichen Polymeren und Stoffen ist erfindungsgemäß möglich, um die Oberflächen der Formkörper die jeweils gewünschten Eigenschaften zu verleihen.

Die Nachbehandlungsschritte können ggf. bei erhöhter Temperatur, jedoch unterhalb der Karbonisierungstemperatur, beispielsweise von 15°C bis 1000°C, vorzugsweise 70°C bis 900°C, besonders bevorzugt 100°C bis 850°C, insbesondere bevorzugt 200°C bis 800°C und insbesondere bei etwa 700 °C durchgeführt werden. In besonders bevorzugten Ausführungsformen werden die erfindungsgemäß hergestellten porösen Formkörper reduktiv oder oxidativ, oder mit einer Kombination dieser Nachbehandlungsschritte bei Raumtemperatur modifiziert.

Durch oxidative bzw. reduktive Behandlung, oder auch den Einbau von Zusatzstoffen, Füllstoffen oder funktionellen Materialien lassen sich die Porendimensionen und deren Eigenschaften bei den erfindungsgemäß hergestellten porösen Formkörpern gezielt beeinflussen bzw. verändern. Beispielsweise können durch Einbau von anorganischen Nanopartikeln oder Nanokompositen wie Schichtsilikaten die Oberflächeneigenschaften des kohlenstoffhaltigen Materials hydrophilisiert oder hydrophobisiert werden.

Weiterhin können die porösen Formkörper durch nachträgliches Beschichten, z.B. mit Polymerlösungen, ein- oder beidseitig verschlossen werden. Diese Beschichtung kann ggf. nochmals karbonisiert werden, um beispielsweise die Stabilität zu erhöhen.

Auch können die erfindungsgemäß hergestellten porösen Formkörper nachträglich durch Einbau geeigneter Zusatzstoffe mit biokompatiblen äußeren und/oder inneren Oberflächen ausgestattet werden. So modifizierte Formkörper lassen sich beispielsweise als Bioreaktoren, Zellkulturträger- bzw. aufzuchtsysteme, Implantate oder als Arzneistoffträger oder -Depots, insbesondere auch in den Körper implantierbare Systeme, eingesetzt werden. Im letzteren Fall können z.B. Medikamente oder Enzyme in das Material eingebracht werden, wobei diese ggf. durch geeignete Retardierung und/oder selektive Permeationseigenschaften aufgebrachter Beschichtungen kontrolliert freigesetzt werden können.

Der poröse Formkörper kann gegebenenfalls auch in einem weiteren optionalen Verfahrensschritt, einem sogenannten CVD-Prozeß (Chemical Vapour Deposition, chemische Gasphasenabscheidung) oder CVI-Prozeß (Chemical Vapour Infiltration) unterzogen werden, um die Oberflächen- oder Porenstruktur und deren Eigenschaften weiter zu modifizieren, ggf. die Poren oberflächlich oder vollständig zu verschließen Hierzu wird die karbonisierte Beschichtung mit geeigneten, Kohlenstoffabspaltenden Precursorgasen bei hohen Temperaturen behandelt. Auch andere Elemente können damit abgeschieden werden, beispielsweise Silizium, Aluminium oder Titan, insbesondere zur Erzeugung der entsprechenden Carbide. Derartige Verfahren sind im Stand der Technik bekannt. Durch entsprechende Vorstrukturierung der Formkörper, beispielsweise unter Verwendung von Fasermaterialien unteschiedlicher Länge und/oder Dicke können so Gradientenwerkstoffe erhalten werden, welche eine über das Volumen des Formkörpers asymmetrisch verteilte Konzentration bestimmter Einlagerungs- oder Reaktionsverbindungen, Beispielsweise der Metall- oder Nichtmetallcarbide, -nitride oder -boride zu erhalten. Man kann somit Gradientenwerkstoffe erhalten, die symmeterisch oder asymetrisch, isotrop oder anisotrop, geschlossenporig, porös oder mit faserartigen Leitstrukturen oder beliebigen Kombinationen hieraus ausgestattet sind.

Als Kohlenstoff-abspaltende Precursor kommen nahezu alle bekannten gesättigten und ungesättigten Kohlenwasserstoffe mit ausreichender Flüchtigkeit unter CVD-Bedingungen in Frage. Beispiele hierfür sind Methan, Ethan, Ethylen, Acetylen, lineare und verzweigte Alkane, Alkene und Alkine mit Kohlenstoffzahlen von C₁ - C₂₀, aromatische Kohlenwasserstoffe wie Benzol, Naphthalin etc., sowie ein- und mehrfach alkyl-, alkenyl- und alkinylsubstituierte Aromaten wie beispielsweise Toluol, Xylol, Cresol, Styrol etc.

Als Keramik-Precursor können BCl₃, NH₃, Silane wie SiH₄, Tetraethoxysilan (TEOS), Dichlorodimethylsilan (DDS), Methyltrichlorosilan (MTS), Trichlorosilyldichloroboran (TDADB), Hexadichloromethylsilyloxid (HDMSO), AlCl₃, TiCl₃ oder Mischungen davon verwendet werden.

Diese Precursor werden in CVD-Verfahren zumeist in geringer Konzentration von etwa 0,5 bis 15 Vol.-% in Mischung mit einem Inertgas, wie beispielsweise Stickstoff, Argon oder dergleichen angewendet. Auch der Zusatz von Wasserstoff zu entsprechenden Abscheidegasgemischen ist möglich. Bei Temperaturen zwischen 500 und 2000°C, vorzugsweise 500 bis 1500°C und besonders bevorzugt 700 bis 1300°C, spalten die genannten Verbindungen Kohlenwasserstofffragmente bzw.
Kohlenstoff oder keramische Vorstufen ab, die sich im Porensystem des porösen Formkörpers im wesentlichen gleichmäßig verteilt niederschlagen, dort die Porenstruktur modifizieren und so zu einer im wesentlichen homogenen Porengröße und Porenverteilung führen.

Mittels CVD-Methoden lassen sich gezielt Poren in dem kohlenstoffhaltigen porösen Formkörper verkleinern, bis hin zur völligen Schließung/Versiegelung der Poren. Hierdurch lassen sich die sorptiven Eigenschaften, wie auch die mechanischen Eigenschaften der Formkörper maßgeschneidert einstellen.

Durch CVD von Silanen oder Siloxanen, gegebenenfalls im Gemisch mit Kohlenwasserstoffen lassen sich die kohlenstoffhaltigen porösen Formkörper durch Carbid- oder Oxycarbidbildung beispielsweise oxidationsbeständig modifizieren. Hierzu sind, sofern sich die Carbide nicht bereits unter CVD-Bedingungen bilden, gegebenenfalls erhöhte Temperaturen nötig, um die Carbidbildung zu fördern.

In bevorzugten Ausführungsformen können die erfindungsgemäßen porösen Formkörper mittels Sputterverfahren zusätzlich beschichtet bzw. modifiziert werden. Hierzu können Kohlenstoff, Silizium oder Metalle bzw. Metallverbindungen aus geeigneter Sputtertargets nach an sich bekannten Verfahren aufgebracht werden. Beispiele hierfür sind Ti, Zr, Ta, W, Mo, Cr, Cu, die in die porösen Formkörper eingestäubt werden können, wobei sich in der Regel die entsprechenden Carbide bilden.

Ferner können mittels Ionenimplantierung die Oberflächeneigenschaften des porösen Formkörpers modifiziert werden. So können durch Implantierung von Stickstoff Nitrid-, Carbonitrid- oder Oxynitridphasen mit eingelagerten Übergangsmetallen gebildet werden, was die chemische Resistenz und mechanische Widerstandsfähigkeit der kohlenstoffhaltigen porösen Formkörper deutlich erhöht.

Die Beschichtung mit z.B. flüssigkristallinen Teerpechen kann zu asymetrischen Werkstoffeigenschaften führen, je nach Ausrichtung der Gitterstrukturen bei der anschließenden Vernetzung, Karbonisierung bzw. Graphitisierung. Dies sind unter anderem die thermische Ausdehnung, die mechanischen Eigenschaften, die elektrische Leitfähigkeit, u.a.

In bestimmten Ausführungsformen kann es vorteilhaft sein, die porösen Formkörper mit einer Beschichtung aus biologisch abbaubaren bzw. resorbierbaren Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymere oder nicht-biologisch abbaubaren bzw. resorbierbaren Polymeren zumindest teilweise zu beschichten. Bevorzugt sind insbesondere anionische, kationischen oder amphotere Beschichtungen, wie z.B. Alginat, Carrageenan, Carboxymethylcellulose; Chitosan, Poly-L-Lysine; und/oder Phosphorylcho lin.

Sofern erforderlich kann in besonders bevorzugten Ausführungsformen der poröse Formkörper nach der Karbonisierung und/oder nach gegebenenfalls erfolgten Nachbehandlungsschritten weiteren chemischen oder physikalischen Oberflächenmodifikationen unterzogen werden. Auch Reinigungsschritte zur Entfernung von eventuellen Rückständen und Verunreinigungen können hier vorgesehen werden. Hierzu können die oben bereits erwähnten Säuren, insbesondere oxidierende Säuren, oder Lösemittel verwendet werden, bevorzugt ist das Auskochen in Säuren oder Lösemitteln.

Die erfindungsgemäßen Formkörper können durch geeignete Wahl der Ausgangs- und Zusatzstoffe in ihrem pH-Wert und der Pufferkapazität in wässriger Umgebung in weiten Bereichen gezielt eingestellt werden. Der pH-Wert erfindungsgemäß hergestellter Formkörper in Wasser kann im Bereich von pH 0 bis pH 14 liegen, vorzugsweise im Bereich von pH 6-8 und besonders bevorzugt bei pH-Werten von 6,5 bis 7,5. Der Pufferbereich erfindungsgemäß hergestellter Formkörper liegt vorzugsweise im neutralen bis sauren Bereich, besonders bevorzugt im schwach sauren Bereich, die Pufferkapazität kann bis zu 50 mol/Liter, vorzugsweise bis zu 10 mol/Liter beträgen und liegt in bevorzugten Anwendungen üblicherweise bei 0,5 bis 5 mol/Liter.

### FORMKÖRPER

Die nach den erfindungsgemäßen Verfahren herstellbaren Formkörper können in beliebigen zwei- oder dreidimensionalen Formen hergestellt werden. Hierzu werden die Halbzeugformteile aus den organischen Polymermaterialien, gegebenenfalls in Mischung mit polymeren oder nichtpolymeren Füllstoffen, mittels geeigneter Formgebungsverfahren zu entsprechenden Rohformen verarbeitet, die gegebenenfalls unter Berücksichtigung des bei der Karbonisierung auftretenden dimensionalen Schwundes den Endformen der porösen kohlenstoffbasierten Formkörper entsprechen. Die erfindungsgemäßen porösen Formkörper können in Form von Rohren, Rundstäben, Platten, Blöcken, Quadern, Würfeln, Voll- oder Hohlkugeln, Flanschen, Dichtungen, Gehäusen und dergleichen hergestellt werden, oder auch länglich geformt sein, wie kreissäulenförmig, mehreckssäulenförmig wie etwa dreieckssäulenförmig oder barrenförmig; oder plattenförmig; oder auch mehreckförmig sein, wie tetraederförmig, pyramidenförmig, oktaederförmig, dodekaederförmig, ikosaederförmig, rhomboid, prismenförmig; oder sphärisch sein, wie etwa kugelförmig, sphärisch oder zylindrisch linsenförmig, oder ringförmig, wabenförmig, mit geraden oder gebogenen Kanälen, gewickelt, gefaltet mit unterschiedlichen Kanaldurchmessern und Durchstömungsrichtungen (parallel, kreuzweise bzw. mit beliebigen Winkeln zwischen den Kanälen).

Gemäß einer besonderen Ausführungsform der vorliegenden Erfindung wird mit einem der erfindungsgemäßen Herstellungsverfahren ein Rohr aus porösem kohlenstoffbasiertem Material hergestellt. Bevorzugt ist dabei die Karbonisierung eines Schlauchs aus Natur- oder Synthesekautschuk oder geeigneten Kunststoffen wie oben als zu Kohlenstoff karbonisierbare kohlenstoffhaltige Formkörper erwähnt, der gegebenenfalls mit Faser- oder Gewebeeinlagen verstärkt ist. Besonders bevorzugt ist die Verwendung eines mit Kunstharzen getränkten Textilgewebes in Form eines Schlauches, das als Halbzeugformteil zur Herstellung eines Rohres aus porösem kunststoffbasierten Material gemäß einem der Verfahren der vorliegenden Erfindung verwendet wird.

Der zur Herstellung eines porösen Rohres verwendete Schlauch kann mehrschichtig aufgebaut sein, beispielsweise umfassend eine innere Schicht aus geschäumtem Kunststoff und eine äußere Schicht aus nichtgeschäumtem Kunststoff oder umgekehrt. Auch die Aufbringung weiterer Schichten ist erfindungsgemäß möglich.

Besonders bevorzugt ist es, dass der mehrschichtige Schlauch durch Koextrusionsblasformen als Halbzeugformteil hergestellt und anschließend zu einem Rohr karbonisiert wird.

In einer weiteren Ausführungsform der vorliegenden Erfindung kann ein Rohr aus kohlenstoffbasiertem Material dadurch hergestellt werden, dass ein mit Polymermaterialien getränktes oder beschichtetes Papiermaterial, beispielsweise auf einer Drehbank zu einem Rohr gewickelt wird, welches anschließend unter Karbonisierungsbedingungen zu einem porösen kohlenstoffhaltigen Rohr karbonisiert wird.

Bevorzugt wird nach diesem Herstellungsverfahren ein flächiges Fasergewebe, Kanalstrukturen oder Filzstrukturen sowie alle Kombinationen hieraus, mit organischen Polymermaterialien imprägniert und/oder beschichtet und über einen geeigneten Dorn aufgewickelt. Anschließend wird mit oder ohne Dorn karbonisiert und der Dorn dann ggf. entfernt. Auf diese Weise lassen sich einfach und präzise poröse Rohre herstellen, die dann noch nachbehandelt, nachverdichtet oder versiegelt werden können.

Durch geeignete Nachbehandlung mittels CVD oder Beschichtung, z.B. mit organischen Polymeren können so hergestellte poröse Rohre ganz oder teilweise versiegelt werden.

Auch ist es erfindungsgemäß möglich, Halbzeugformteile zur Herstellung von Rohren wie Polymerschläuche, insbesondere auch Endlosschläuche in kontinuierlichen Verfahren zur Herstellung von Kohlenstoffrohren zu verwenden. Besonders bevorzugt ist auch hier die Verwendung faserverstärkter Schläuche, wobei die Fasern ausgewählt werden können aus Textil oder Gewebefasern, Glasfasern, Kohlefasern, Steinwolle, Polymerfasern, beispielsweise aus Polyacrylnitril, Nonwoven-Materialien, Faservliese, Filze, Cellulose, PET-Fasern und beliebige Mischungen dieser Materialien.

Durch Verwendung mehrschichtiger Halbzeugformteile können asymmetrische Aufbauten der erfindungsgemäß herstellbaren kohlenstoffhaltigen Formkörper realisiert werden. Beispielsweise können geschäumte Polymermaterialien wie Polyurethanschaum, Polyacrylnitrilschaum und dergleichen mit einer weiteren Schicht aus dichtem Polymermaterial geformt werden, die anschließend zu Formkörpern mit regional unterschiedlicher Porositätsverteilung karbonisiert werden.

Bei Hohlkörpern können bereits im Halbzeugformteil Flansche mit anlaminiert werden, die dann im Wesentlichen geschlossenporig durchkarbonisiert werden. Beim Einsatz von Polymerfasern und -Geweben entstehen so Vollkarbon-Moduleinheiten mit ausgezeichnetem Haftverbindung zwischen Faser und Matrix.

Die nach einem erfindungsgemäßen Verfahren hergestellten kohlenstoffbasierten Formkörper, insbesondere Kohlenstoffrohre lassen sich als Rohrmembran, in Rohrmembranreaktoren, in Rohrbündelreaktoren und Wärmeaustauschern wie auch in Bioreaktoren verwenden.

Auch als poröse Katalysatorträger, insbesondere im Automobilbereich oder der Rauchgasreinigung in technischen Anlagen, sind die erfindungsgemäßen Formkörper verwendbar. Vorteilhaft ist hierbei ihre Hitzebeständigkeit, ihre chemische Widerstandsfähigkeit und Formstabilität. Ferner sind die erfindungsgemäßen Formkörper und Materialien nahezu spannungsfrei und extrem thermoschockstabil, d.h. auch starke Temperatursprünge werden problemlos vertragen. Durch Aufbringung von Metallen, insbesondere von Edelmetallen, und sonstigen katalytisch aktiven Materialien lassen sich langzeitstabile und hocheffektive Katalysatorträger nach den erfindungsgemäßen Verfahren herstellen.

Aus flächigen Kanalstrukturen hergestellte Platten, sowie hieraus gewickelte Rohrstrukturen eignen sich ausgezeichnet als Isoliermaterialien, z.B. für Hochtemperaturanwendungen bzw. zur Abschirmung von Mikrowellen (Mikrowellwnabsorber). Die elektrischen Eigenschaften sind hierbei derart einstellbar, dass z.B. Hochfrequenzheizungen ihre Energie nahezu verlustfrei durch diese Isoliermaterialien in den Ofenbereich einkoppeln können. Hochorientierte Werkstoffe können aber auch so eingestellt werden, dass sie direkt durch Hochfrequenz angeregt und somit direkt beheizt werden. Dies ist auch ein einfaches Verfahren zur technischen Herstellung (Karbonisierung) bzw. zur Graphitisierung.

Nach den erfindungsgemäßen Verfahren hergestellte Formkörper können auch als medizinische Implantate, beispielsweise orthopädische, chirurgische, und/oder nichtorthopädische Implantate wie Knochen- oder Gelenkprothesen, orthopädische Platten, Schrauben, Nägel, Fixierungen und dergleichen verwendet werden.

Aufgrund Ihrer Biokompatibilität und der vielfältig einstellbaren Oberflächeneigenschaften wie Adsorptionsfähigkeit, Absorptionsfähigkeit, Adhäsion von biologischem Material, in weiten Bereichen spezifisch einstellbarer Porosität, der Porengrößen und -volumina bis hin zu geschlossenporigen Formkörpern etc. ist die Verwendung der erfindungsgemäß herstellbaren Formkörper als Substrat bzw. Träger für die Besiedlung mit Mikroorganismen und Zellkulturen besonders bevorzugt.

Insbesondere bevorzugt ist die Verwendung der erfindungsgemäß hergestellten kohlenstoffbasierten, kohlenstoffhaltigen Formkörper sowie keramischen Materialien und Komposita als Träger- und/oder Aufzuchtsysteme (TAS) für die Kultivierung primärer Zellkulturen wie eukaryote Gewebe, z.B. Knochen, Knorpel, Leber, Nieren, sowie zur Kultivierung bzw. Immobilisierung von xenogenen, allogenen, syngenen oder autologen Zellen und Zelltypen, sowie gegebenenfalls auch von genetisch modifizierte Zelllinien.

Neben den erfindungsgemäß herstellbaren Formkörpern sind hierzu prinzipiell alle porösen oder nichtporösen, kohlenstoffhaltigen Materialien geeignet zur Verwendung als Träger- und Aufzuchtsysteme (TAS) für die Kultivierung primärer Zellkulturen. Bevorzugt ist neben den erfindungsgemäß herstellbaren Formkörpern auch die Verwendung von Materialien wie sie in der WO 02/32558 ("Flexible, poröse Materialien und Adsorbentien... ") beschrieben sind, deren Offenbarung hiermit vollständig einbezogen wird, insbesondere die auf den Seiten 24, Zeile 11 bis Seite 43 beschriebenen Kohlenstoff- und Keramik-Materialien, Membranen und Träger. Auch symmetrische oder asymmetrische, texturierte kohlenstoff- oder keramikbasierte Materialien und deren Kombinationen sind zur Verwendung als TAS geeignet.

Die genannten Materialien und Formkörper können speziell auch als Träger- und Aufzuchtsysteme für Nervengewebe verwendet werden. Besonders vorteilhaft ist, dass kohlenstoffhaltige Materialien hier insbesondere durch die einfache Einstellung der Leitfähigkeit der Formkörper und die Applikation von Impulsströmen zur Kultivierung von Nervengewebe besonders anpassbar und geeignet sind.

Die genannten Materialien und Formkörper dienen in der erfindungsgemäßen Verwendung als TAS ferner auch als in vitro- oder in vivo-Leitstrukturen, sog. Scaffolds, für 2- und 3-dimensionales Gewebewachstum; durch deren spezifische Formgebung ist es möglich, aus Zellkulturen Organteile oder ganze Organe zu züchten. Die TAS unterstützen bzw. modulieren hierbei als Leitstruktur durch geeignete Einstellung der Porosität, durch das Flow-Channel-Design und die zwei- bzw. dreidimensionale Formgebung das Zell-, Gewebe- bzw. Organwachstum in physikalischer Hinsicht, insbesondere aber auch durch einstellbare Bereitstellung, Verteilung und Nachschub von Nährlösung bzw. -medium am Verbrauchsort, sowie durch Unterstützung bzw. Förderung der Zell- und Gewebsproliferation und - Differenzierung.

Für die Verwendung als TAS können die Materialien und Formkörper bzw. Trägersysteme 2- und 3-dimensional geformt sein. Geeignete Makrostrukturen sind beispielsweise Rohre, insbesondere für die Produktion bzw. Züchtung natürlicher Gefäße, kubische Formen, etc, wie oben bei Formkörpern erwähnt.

Insbesondere können die erfindungsgemäßen Formkörper und andere kohlenstoffbasierte Materialien für die Verwendung als TAS natürlichen Organformen nachempfunden werden, z.B. knorpelige Gelenkflächen von Knie-, Hüft-, Schulter-, Fingergelenken, etc. die dann zur Aufzucht entsprechend geformter Knorpel, Knochenhäute und dergleichen verwendet werden können. Diese können dann entweder mit dem aufgewachsenem Gewebe implantiert werden, oder das gezüchtete Gewebe wirddurch Methoden des Standes der Technik wie z.B. mechanische oder chemisch-enzymatische Ablösung, in entsprechend gewachsener Form abgetrennt und dann implantiert.

Da kohlenstoffbasierte Materialien und Formkörper auch gute mechanische Eigenschaften aufweisen, die deren Verwendung als Implantate, z.B. als künstliche Gelenke und dergleichen möglich machen, können diese erfindungsgemäß in einer Gewebekultur als Substrate oder Träger verwendet werden, und nach dem Aufwachsen einer ausreichenden Knorpelschicht als hochverträgliche, biomimetische Implantate in den Körper von Patienten eingesetzt werden. So ist es erfindungsgemäß möglich, einzelnen Patienten Implantate einzusetzen, die mit körpereigenem Gewebe beschichtet sind, das aus geeigneten Zellproben des Patienten direkt auf dem Implantat gezüchtet wurde. Hierdurch lassen sich Abstoßungsphänomene und Immunabwehrreaktionen vermindern bzw. vollständig vermeiden.

Erfindungsgemäß können die Formkörper und Materialien als TAS zur Kultivierung in existierenden Bioreaktorsystemen verwendet werden, z. B. passive Systeme ohne kontinuierliche Regeltechnikwie z.B. Gewebeplatten, Gewebeflaschen, Roller-Bottles; aber auch aktive Systeme mit Gaszufuhr und automatischer Einstellung von Parametern (Azidität, Temperatur), also im weitesten Sinne Reaktorsysteme mit Mess- und Regeltechnik.

Ferner können die erfindungsgemäßen TAS durch Vorsehung geeigneter Vorrichtungen wie z.B. Anschlüsse für die Perfusion mit Nährlösungen und den Gasaustausch als Reaktorsystem betrieben werden insbesondere auch modular in entsprechenden Reihenreaktorsystemen und Gewebekulturen.

Erfindungsgemäße TAS können darüber hinaus als ex vivo Reaktorsysteme, z.B. extrakorporale Assistenzsysteme, oder als Organreaktoren verwendet werden, z.B. sogenannten liver assist systems oder liver replacement systems; oder auch in vivo oder in vitro für verkapselte (engl. encapsulated) Inselzellen, z. B. als künstl. Pankreas, verkapselte Urothelzellen, z.B. als künstl. Niere und dergleichen, die vorzugsweise implantierbar sind.

Darüber hinaus können die erfindungsgemäßen TAS zur Förderung der Organogenese geeignet modifiziert werden, beispielsweise mit Proteoglykanen, Kollagenen, gewebetypischen Salzen, z.B. Hydroxylapatit etc., besonders auch mit den obengenannten biologisch abbaubaren bzw. resorbierbaren Polymeren.

Bevorzugt werden die erfindungsgemäßen TAS ferner durch Imprägnierung und/oder Adsorption von Wachstumsfaktoren, Cytokinen, Interferone und/oder Adhäsionsfaktoren modifiziert. Beispiele geeigneter Wachstumsfaktoren sind PDGF, EGF, TGF-α, FGF, NGF, Erythropoietin, TGF-β, IGF-I und IGF-II. Geeignete Cytokine umfassen beispielsweise IL-1-α und -β, IL-2, IL-3, IL-4, IL-5, IL-6, IL-7, IL-8, IL-9, IL-10, IL-11, IL-12, IL-13. Geeignete Interferone umfassen z.B . INF-α und -β, INF-γ. Beispiele geeigneter Adhäsionsfaktoren sind Fibronectin, Laminin, Vitronectin, Fetuin, Poly-D-Lysin und dergleichen.

Ferner sind die erfindungsgemäßen Formkörper, insbesondere bei Verwendung als TAS auch anwendbar als Microarray-Systeme für z.B. Drug Discovery, Tissue screening, Tissue engineering etc.

### BEISPIELE

Die folgenden Beispiele dienen der Veranschaulichung der erfindungsgemäßen Prinzipien und sind nicht einschränkend gedacht.

### Beispiel 1:

Zur Herstellung eines Rohres im Wickelverfahren mit DN25 Kern, Länge 500mm, Wandstärke 3 mm, wurde ein Glasfasergewebe aus E-CR-Glas (chemikalienbeständig modifiziertes E-Glas) von 30mm Breite, beschichtet/imprägniert mit GFK-Harz auf Phenolharzbasis im Kreuzgang auf einem entsprechenden Stahldorn verlegt und der Dorn entfernt. Das Gewicht betrug 3,6 g/cm vor der Pyrolyse. Die Pyrolyse wurde unter Stickstoff bei 800°C über 48 Stunden durchgeführt. Das Gewicht nach der Pyrolyse betrug 3,0 g/cm. Die Membraneigenschaften wurden mit dem Bubble-Point Test vermessen (ASTM E1294), wobei eine Porengröße von 500 Angström ermittelt wurde.

### Beispiel 2:

Rohrherstellung im Wickelverfahren wie unter Beispiel 1 angegeben, unter Verwendung eines Glasfaservlieses aus C-Glas (chemikalienbeständiges C-Glas, nonwoven) mit 30mm Breite und GFK-Harz auf Vinylesterharzbasis, Verlegung auf Stahldorn im Kreuzgang. Gewicht 3,5 g/cm vor der Pyrolyse. Pyrolyse unter Stickstoff bei 800°C über 48 Stunden. Gewicht nach der Pyrolyse 0,9 g/cm. Die Membraneigenschaften wurden mit dem Bubble-Point Test vermessen (ASTM E1294) und eine Porengröße von 0,8 Mikrometern ermittelt.

### Beispiel 3:

Rohrherstellung im Wickelverfahren wie unter Beispiel 1 angegeben, unter Verwendung eines Polyacrylnitril (PAN) nonwoven (Fa. Freudenberg) von 30mm Breite und GFK-Harz auf Phenolharzbasis, Verlegung auf Stahldorn im Kreuzgang. Gewicht 3,5 g/cm vor der Pyrolyse. Pyrolyse unter Stickstoff bei 800°C über 48 Stunden. Gewicht nach der Pyrolyse 1,94 g/cm. Die Membraneigenschaften wurden mit dem Bubble-Point Test vermessen (ASTM E1294). Es konnte im Messbereich keine Porengröße (Gasdurchbruch) festgestellt werden. Anschließende Teiloxidation im Luftstrom bei 400 °C für 15 Minuten lieferte eine durchschnittliche Porengröße nach Bubble-Point Test von 1,2 µm.

### Beispiel 4:

Rohrherstellung im Wickelverfahren wie unter Beispiel 1 angegeben, unter Verwendung eines Glasfaservlieses aus E-CR-Glas (chemikalienbeständig modifiziertes E-Glas) von 30 mm Breite, und Polyacrylnitril (PAN) nonwoven (Fa. Freudenberg) von 30mm Breite (Verhältnis 1:1) und GFK-Harz auf Phenolharzbasis, Verlegung auf Stahldorn im Kreuzgang. Gewicht 3,6g/cm vor der Pyrolyse. Pyrolyse unter Stickstoff bei 800°C über 48 Stunden. Gewicht nach der Pyrolyse 2,0 g/cm.

### Beispiel 5:

Rohrherstellung im Wickelverfahren wie unter Beispiel 1 angegeben, unter Verwendung eines Glasfaservlieses aus E-CR-Glas (chemikalienbeständig modifiziertes E-Glas) von 30 mm Breite, und Polyacrylnitril (PAN) nonwoven (Fa. Freudenberg) von 30mm Breite (Verhältnis 1:1) und GFK-Harz auf Phenolharzbasis mit 20 % Aerosil R972 Verlegung auf Stahldorn im Kreuzgang. Gewicht 3,6g/cm vor der Pyrolyse. Pyrolyse unter Stickstoff bei 800°C über 48 Stunden. Gewicht nach der Pyrolyse 3,0 g/cm. Anschließend wird mit 30%iger NaOH Lauge das Aerosil ausgewaschen. Die Membraneigenschaften wurden mit dem Bubble-Point Test vermessen (ASTM E1294), wobei eine Porengröße von 0,6 µm ermittelt wurde.

### Beispiel 6:

Es wurden kohlenstoffbasierte Platten aus naturfaserverstärktem, Komposit-Polymer mit anorganischen Füllstoffen und mit einem Flächengewicht von 100g/m² und 110 Mikrometern Dicke hergestellt. Dieses flächige Kompositmaterial wurde duch eine handelsübliche Prägemaschine mit einer Kanalstruktur versehen, die einen Kanaldurchmesser nach dem Aufeinanderlegen zweier Blätter vom 3 mm ergab. Diese Blätter wurden zu wabenförmigen Blöcken verklebt und unter Schutzgas (Stickstoff) bei 800°C über 48 Stunden karbonisiert. Der Druckverlust in Kanalrichtung betrug nur 0,1 bar/m, es ergab sich bei der Karbonisierung ein Gewichtsverlust von 66 Gew.-%.
Ein aus diesem Material gewickeltes Rohr von 10cm Länge und 40mm Durchmesser bei einer Wandstärke von 6mm wurde in einem Einkoppelversuch in einer 8 KHz Hochfrequenzheizeinrichtung eingestellt. Der Ruhestrom änderte sich praktisch nicht im Vergleich zum Ruhestrom und es erfolgt auch nach 5 Minuten keine nennenswerte Erwärmung der Werkstoffs. Das derartig hergestellt Material lässt sich problemlos und präzise sägen, bohren, Fräsen, etc.

### Beispiel 7:

Für die vorgesehene Anwendung als Trägermaterial für Zellkulturaufzuchtsysteme wurde ein naturfaserhaltiges Polymerkomposit mit einem Flächengewicht von 100 g/m² und 110 µm Dicke in einer Stickstoffatmosphäre bei 800 °C über 48 Stunden karbonisiert, wobei gegen Ende Luft zugesetzt wurde, um die Poren zu modifizieren. Es trat ein Gewichtsverlust von 50 Gew.-% auf. Das resultierende Material weist in Wasser einen pH-Wert von 7,4 und einen Pufferbereich im schwach Sauren auf. 20x40 mm große Stücke von je etwa 60 µm Dicke dieses Kohlenstoffmaterials wurden auf üblichen Sechser-Gewebeplatten mit 4 ml Nährlösung und je 1,5 ml Zellsuspension beschickt. Die Zellsuspension enthielt Hybridoma FLT₂ MAB gegen Shigatoxin produzierende Zellinien, bekannt für nicht adhärentes, nicht adhäsives suspensionsständiges Wachstum.
Als Vergleich wurden Sechser-Gewebeplatten ohne Kohlenstoffmaterial bei sonst gleichen Bedingungen und Beschickung verwendet.
Die Proben mit erfindungsgemäßem Träger zeigten eine spontane, quantitative Immobilisierung der Zellen, es konnte keine Trübung der Suspension mehr nachgewiesen werden. Innerhalb von 7 Tagen Inkubationszeit kam es zu einer versiebenfachung der Zelldichte auf 1,8 x 10⁷ Zellen pro ml. Die MAB Produktion stieg von anfänglich 50 µg/ml auf 350 µl/ml der durchschnittlichen Kulturlebensdauer, ohne Zeichen eines proteolytischen Abbaus. 12 von 12 Proben waren nach 25 Tagen noch lebend, wonach abgebrochen wurde. Dies zeigt, dass die erfindungsgemäßen Träger zu einer Unterbrechung der Kontaktinhibition trotz der höheren Zelldichte führen. Selbst nach Kryokonservierung und Auftauen erfolgen nach Zugabe frischen Nährmediums ein spontanes Wiedereinsetzen der MAB-Produktion.

Im Vergleichsexperiment hat nur eine von 6 Kulturen bis zum 11. Tag überlebt.

Die Anmeldung beinhaltet darüber hinaus die folgenden Ausführungsformen:
1. Verfahren zur Herstellung von porösen kohlenstoffbasierten Formkörpern,
   **gekennzeichnet durch** folgende Schritte:
   - Mischen von zu Kohlenstoff karbonisierbaren organischen Polymermaterialien mit nichtpolymeren Füllstoffen;
   - Herstellen eines Halbzeugformteils aus der Mischung;
   - Karbonisieren des Halbzeugformteils in nichtoxidierender Atmosphäre bei erhöhter Temperatur, wobei ein kohlenstoffbasierter Formkörper erhalten wird;
   - Herauslösen der Füllstoffe aus dem karbonisierten Formkörper mit geeigneten Lösemitteln.
2. Verfahren nach Ausführungsform 1,
   **dadurch gekennzeichnet, dass** die Füllstoffe ausgewählt sind aus anorganischen Metallsalzen, insbesondere der Salze von Alkali- und/oder Erdalkalimetallen, vorzugsweise Alkali- oder Erdalkalicarbonate, -sulfate, -sulfite, -nitrate, -nitrite, - phosphate, -phosphite, -halogenide, -sulfide, -oxide, sowie Mischungen dieser.
3. Verfahren nach Ausführungsform 1 oder 2,
   **dadurch gekennzeichnet, dass** die Füllstoffe ausgewählt sind aus organischen Metallsalzen, vorzugsweise solche der Alkali-, Erdalkali- und/oder Übergangsmetalle, insbesondere deren Formiate, Acetate, Propionate, Malate, Maleate, Oxalate, Tartrate, Citrate, Benzoate, Salicylate, Phthalate, Stearate, Phenolate, Sulfonate, Aminsalze, sowie Mischungen dieser.
4. Verfahren nach einer der vorhergehenden Ausführungsformen,
   **dadurch gekennzeichnet, dass** zum Herauslösen der Füllstoffe Wasser oder verdünnte oder konzentrierte, anorganische oder organische Säuren verwendet werden.
5. Verfahren nach einer der Ausführungsformen 1 bis 3,
   **dadurch gekennzeichnet, dass** zum Herauslösen der Füllstoffe organische Lösungsmittel verwendet werden.
6. Verfahren nach einer der vorhergehenden Ausführungsformen,
   **dadurch gekennzeichnet, dass** die verwendeten Füllstoffe aus Substanzen bestehen, die während des Karbonisierungsschrittes in eine lösliche Form umgewandelt werden.
7. Verfahren zur Herstellung von porösen kohlenstoffbasierten Formkörpern,
   **gekennzeichnet durch** folgende Schritte:
   - Mischen von zu Kohlenstoff karbonisierbaren organischen Polymermaterialien mit polymeren Füllstoffen;
   - Herstellen eines Halbzeugformteils aus der Mischung;
   - Karbonisieren des Halbzeugformteils in nichtoxidierender Atmosphäre bei erhöhter Temperatur, wobei die polymeren Füllstoffe im Wesentlichen vollständig abgebaut werden.
8. Verfahren nach Ausführungsform 7,
   **dadurch gekennzeichnet, dass** die polymeren Füllstoffe ausgewählt sind aus gesättigten, verzweigten oder unverzweigten aliphatischen Kohlenwasserstoffhomo- oder -copolymeren, vorzugsweise Polyolefine wie Polyethylen, Polypropylen, Polybuten, Polyisobuten, Polypenten, sowie Mischungen davon.
9. Verfahren nach einer der Ausführungsformen 7 oder 8
   **dadurch gekennzeichnet, dass** der Formkörper nach der Karbonisierung mit Oxidations- oder Reduktionsmitteln behandelt wird.
10. Verfahren zur Herstellung von porösen kohlenstoffbasierten Formkörpern,
   **gekennzeichnet durch** folgende Schritte:
   - Herstellen eines Halbzeugformteils aus karbonisierbaren organischen Polymermaterialien;
   - Karbonisieren des Halbzeugformteils in nichtoxidierender Atmosphäre bei erhöhter Temperatur, wobei ein kohlenstoffbasierter Formkörper erhalten wird; und
   - Teiloxidation des karbonisierten Formkörpers zur Erzeugung von Poren.
11. Verfahren nach Ausführungsform 10,
   **dadurch gekennzeichnet, dass** die Teiloxidation mittels Wärmebehandlung in oxidierender Gasatmosphäre erfolgt.
12. Verfahren nach Ausführungsform 11,
   **dadurch gekennzeichnet, dass** die Teiloxidation mittels Luft, Sauerstoff, Kohlenmonoxid, Kohlendioxid, Stickstoffoxiden bei Temperaturen m Bereich von 50 °C bis 800 °C durchgeführt wird.
13. Verfahren nach Ausführungform 10,
   **dadurch gekennzeichnet, dass** die Teiloxidation mit oxidierenden Säuren erfolgt.
14. Verfahren nach einer der vorhergehenden Ausführungsformen,
   **dadurch gekennzeichnet, dass** das karbonisierbare organische Polymermaterial ungesättigte, verzweigte aliphatische Kohlenwasserstoffe, verzweigte oder unverzweigte, vernetzte oder unvernetzte aromatische oder teilaromatische Kohlenwasserstoffe, sowie substituierte Derivate davon umfasst.
15. Verfahren nach einer der vorhergehenden Ausführungsformen,
   **dadurch gekennzeichnet, dass** das karbonisierbare organische Polymermaterial ausgewählt ist aus Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phtalat), Alkydharz, Chlorkautschuk, Epoxidharz, Acrylatharz, Phenolharz, Aminharz, Melaminharz, Alkylphenolharze, epoxidierte aromatische Harze, Teer, teerartige Materialien, Teerpech, flüssigkristaline Teerpeche, Bitumen, Stärke, Zellulose, Schellack, Fasern aus Polyacrylnitril, Cellulose oder Novolak, organische Materialien aus nachwachsenden Rohstoffen sowie deren Copolymere, Mischungen und Kombinationen dieser Homo- oder Copolymere.
16. Verfahren nach einer der vorhergehenden Ausführungsformen,
   **dadurch gekennzeichnet, dass** das Polymermaterial übliche Additive wie Füllstoffe, Weichmacher, Gleitmittel, Flammschutzmittel, Glas, Glasfasern, Kohlefasern, Baumwolle, Gewebe, Metallpulver, Metallverbindungen, Metalloxide, Silizium, Siliziumoxid, Zeolithe, TiO₂, Aluminiumoxide, Aluminosilikate, Zirkonoxide, Talkum, Graphit, Russ, Tonmaterialien, Phyllosilikate enthält.
17. Verfahren nach einer der vorhergehenden Ausführungsformen,
   **dadurch gekennzeichnet, dass** die Herstellung der Halbzeugformteile mittels Guss-, Extrusions-, Press-, Spritzguss- oder anderen üblichen Formgebungsverfahren erfolgt.
18. Verfahren nach einer der vorhergehenden Ausführungsformen,
   **dadurch gekennzeichnet, dass** die Karbonisierung unter Schutzgas, vorzugsweise Stickstoff oder Argon, ggf. bei Unterdruck, oder im Vakuum, ggf. unter Zumischung reaktiver Gase wie Wasserstoff, bei Temperaturen im Bereich von 200°C bis 4000°C durchgeführt wird.
19. Verfahren nach einer der vorhergehenden Ausführungsformen,
   ferner umfassend den Schritt des Abscheidens von Kohlenstoff, Stickstoff, Silizium und/oder von Metallen mittels chemischer oder physikalischer Dampfabscheidung (CVD bzw. PVD), Sputtern, Ionenimplantierung oder chemischer Dampfinfiltration (CVI) auf der Oberfläche des Formkörpers und/oder in dessen Poren.
20. Verfahren nach Ausführungsform 19,
   **dadurch gekennzeichnet, dass** die Poren des Formkörpers ganz oder teilweise versiegelt werden.
21. Poröser Formkörper,
   herstellbar nach dem Verfahren nach einem der vorhergehenden Ansprüche.
22. Formkörper nach Ausführungsform 21,
   in Form von Rohren, Rundstäben, Platten, Blöcken, Quadern, Würfel, Spritzgussformen, Wabenstrukturen, geprägten, gefalteten, gewickelten, gerollten zwei-oder dreidimensionalen Strukturen, mit Kanalstrukturen, Voll- oder Hohlkugeln, Flanschen, Dichtungen, Gehäusen und dergleichen.
23. Rohr,
   herstellbar nach dem Verfahren nach einer der Ausführungsformen 1 bis 20, aus einem Schlauch aus Natur- oder Synthese-Kautschuk, Cellulose, Epoxidharzmasse oder Kunststoffen, ggf. verstärkt mit Faser- oder Gewebeeinlagen.
24. Rohr nach Ausführungsform 23,
   **dadurch gekennzeichnet, dass** als Schlauch ein Textilgewebe verwendet wird, das mit Kunstharzen getränkt ist.
25. Rohr nach Ausführungsform 23,
   **dadurch gekennzeichnet, dass** ein mehrschichtiger Schlauch carbonisiert wird.
26. Rohr nach Ausführungsform 25,
   **dadurch gekennzeichnet, dass** der mehrschichtige Schlauch eine innere Schicht aus geschäumtem Kunststoff und eine äußere Schicht aus nichtgeschäumtem Kunststoff enthält.
27. Rohr nach einer der Ausführungsformen 25 oder 26,
   **dadurch gekennzeichnet, dass** der mehrschichtige Schlauch durch Koextrusionsblasformen erhalten wurde.
28. Katalysatorträger, herstellbar nach einer der Ausführungsformen 1 bis 22.
29. Isoliermaterial, herstellbar nach einer der Ausführungsformen 1 bis 22.
30. Verwendung des Rohrs nach einer der Ausführungsformen 23 bis 27,
   als Rohrmembran, in Rohrmembranreaktoren, Rohrbündelreaktoren, in Wärmeaustauschern, zur Destillation, Pervaporation, Rückgewinnung und/oder Kreislaufführung von Reaktionsprodukten und/oder Extraktion in hierzu geeigneten Vorrichtungen.
31. Verwendung des Formkörpers nach Ausführungsform 21 oder 22 als Träger und/oder Aufzuchtsystem für die Kultivierung primärer Zellkulturen.
32. Verwendung nach Ausführungsform 31, wobei die Zellkulturen ausgewählt sind aus eukaryoten Geweben wie Knochen, Knorpel, Leber, Nieren, Pankreas, Nerven und dergleichen, sowie xenogenen, allogenen, syngenen oder autologen Zellen und Zelltypen, sowie aus genetisch modifizierten Zelllinien.
33. Verwendung nach Ausführungsform 31 oder 32, wobei der Formkörper als Leitstruktur für zwei- oder dreidimensionales Gewebewachstum, insbesondere zu Züchten von Organen oder Organteilen verwendet wird.
34. Verwendung nach einer der Ausführungsformen 31 bis 33,
   **dadurch gekennzeichnet, dass** das Träger- und/oder Aufzuchtsystem ex vivo als Reaktorsystem verwendet wird.
35. Verwendung nach einer der Ausführungsformen 31 bis 33,
   **dadurch gekennzeichnet, dass** das Träger- und/oder Aufzuchtsystem in vivo als Implantat eingesetzt wird.
36. Verwendung nach einer der Ausführungsformen 31 bis 35,
**dadurch gekennzeichnet, dass** das Träger- und/oder Aufzuchtsystem mit Proteoglykanen, Kollagenen, gewebetypischen Salzen, oder biologisch abbaubaren bzw. resorbierbaren Polymeren modifiziert ist.

## Patentansprüche

1. Verfahren zur Herstellung von porösen kohlenstoffbasierten Formkörpern,
**gekennzeichnet durch** folgende Schritte:
- Herstellen eines Halbzeugformteils aus karbonisierbaren organischen Polymermaterialien;
- Karbonisieren des Halbzeugformteils in nichtoxidierender Atmosphäre bei erhöhter Temperatur, wobei ein kohlenstoffbasierter Formkörper erhalten wird; und
- Teiloxidation des karbonisierten Formkörpers zur Erzeugung von Poren.

2. Verfahren nach Anspruch 1,
**dadurch gekennzeichnet, dass** die Teiloxidation mittels Wärmebehandlung in oxidierender Gasatmosphäre erfolgt.

3. Verfahren nach Anspruch 2,
**dadurch gekennzeichnet, dass** die Teiloxidation mittels Luft, Sauerstoff, Kohlenmonoxid, Kohlendioxid, Stickstoffoxiden bei Temperaturen im Bereich von 50 °C bis 800 °C durchgeführt wird.

4. Verfahren nach Anspruch 1,
**dadurch gekennzeichnet, dass** die Teiloxidation mit oxidierenden Säuren erfolgt.

5. Verfahren nach einem der vorhergehenden Ansprüche,
**dadurch gekennzeichnet, dass** das karbonisierbare organische Polymermaterial ungesättigte, verzweigte aliphatische Kohlenwasserstoffe, verzweigte oder unverzweigte, vernetzte oder unvernetzte aromatische oder teilaromatische Kohlenwasserstoffe, sowie substituierte Derivate davon umfasst.

6. Verfahren nach einem der vorhergehenden Ansprüche,
**dadurch gekennzeichnet, dass** das karbonisierbare organische Polymermaterial ausgewählt ist aus Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phtalat), Alkydharz, Chlorkautschuk, Epoxidharz, Acrylatharz, Phenolharz, Aminharz, Melaminharz, Alkylphenolharze, epoxidierte aromatische Harze, Teer, teerartige Materialien, Teerpech, flüssigkristaline Teerpeche, Bitumen, Stärke, Zellulose, Schellack, Fasern aus Polyacrylnitril, Cellulose oder Novolak, organische Materialien aus nachwachsenden Rohstoffen sowie deren Copolymere, Mischungen und Kombinationen dieser Homo- oder Copolymere.

7. Verfahren nach einem der vorhergehenden Ansprüche,
**dadurch gekennzeichnet, dass** das Polymermaterial Additive wie Füllstoffe, Weichmacher, Gleitmittel, Flammschutzmittel, Glas, Glasfasern, Kohlefasern, Baumwolle, Gewebe, Metallpulver, Metallverbindungen, Metalloxide, Silizium, Siliziumoxid, Zeolithe, TiO₂, Aluminiumoxide, Aluminosilikate, Zirkonoxide, Talkum, Graphit, Russ, Tonmaterialien, Phyllosilikate enthält.

8. Verfahren nach einem der vorhergehenden Ansprüche,
**dadurch gekennzeichnet, dass** die Herstellung der Halbzeugformteile mittels Guss-, Extrusions-, Press-, Spritzguss- oder anderen üblichen Formgebungsverfahren erfolgt.

9. Verfahren nach einem der vorhergehenden Ansprüche,
**dadurch gekennzeichnet, dass** die Karbonisierung unter Schutzgas, vorzugsweise Stickstoff oder Argon, ggf. bei Unterdruck, oder im Vakuum, ggf. unter Zumischung reaktiver Gase wie Wasserstoff, bei Temperaturen im Bereich von 200°C bis 4000°C durchgeführt wird.

10. Poröser Formkörper,
herstellbar nach dem Verfahren nach einem der vorhergehenden Ansprüche.

11. Formkörper nach Anspruch 10,
in Form von Rohren, Rundstäben, Platten, Blöcken, Quadern, Würfel, Spritzgussformen, Wabenstrukturen, geprägten, gefalteten, gewickelten, gerollten zwei-oder dreidimensionalen Strukturen, mit Kanalstrukturen, Voll- oder Hohlkugeln, Flanschen, Dichtungen, Gehäusen und dergleichen.

12. Verwendung des Formkörpers nach Anspruch 10 oder 11 als Träger und/oder Aufzuchtsystem für die Kultivierung primärer Zellkulturen.

13. Verwendung nach Anspruch 12, wobei die Zellkulturen ausgewählt sind aus eukaryoten Geweben wie Knochen, Knorpel, Leber, Nieren, Pankreas, Nerven und dergleichen, sowie xenogenen, allogenen, syngenen oder autologen Zellen und Zelltypen, sowie aus genetisch modifizierten Zelllinien.

14. Verwendung nach Anspruch 12 oder 13, wobei der Formkörper als Leitstruktur für zwei- oder dreidimensionales Gewebewachstum, insbesondere zu Züchten von Organen oder Organteilen verwendet wird.

15. Verwendung nach einem der Ansprüche 12 bis 14,
**dadurch gekennzeichnet, dass** das Träger- und/oder Aufzuchtsystem ex vivo als Reaktorsystem verwendet wird.

16. Verwendung nach einem der Ansprüche 12 bis 14,
**dadurch gekennzeichnet, dass** das Träger- und/oder Aufzuchtsystem in vivo als Implantat eingesetzt wird.

17. Verwendung nach einem der Ansprüche 12 bis 16,
**dadurch gekennzeichnet, dass** das Träger- und/oder Aufzuchtsystem mit Proteoglykanen, Kollagenen, gewebetypischen Salzen, Wachstumsfaktoren, Gelatine, oder biologisch abbaubaren bzw. resorbierbaren Polymeren oder beliebigen Mischungen davon modifiziert ist.
